# EUROPEAN PATENT APPLICATION

(11) **EP 4 344 873 A1**
(43) Date of publication of application: **03.04.2024**
(21) Application number: 22020459.8
(22) Date of filing: 27.09.2022
(51) Int. Cl.: B33Y 40/20, C09D 183/04

(54) **POST-TREATMENT OF A 3D-PRINTED ELASTOMER SILICONE ARTICLE**

(71) Applicant: Elkem Silicones France SAS, 69003 Lyon (FR); Elkem Silicones USA Corp., East Brunswick, NJ 08816-1100 (US)
(72) Inventor: The designation of the inventor has not yet been filed
(74) Representative: Menville, Laure

(57) **Abstract**

The present invention relates to a method of post-treatment of a 3D-printed silicone article, said method comprising the coating the 3D-printed silicone article with a crosslinkable silicone composition comprising a microfiller. The invention further relates to a crosslinkable silicone composition comprising a microfiller, and its use as post-treatment composition for the post-treatment of a 3D printed silicone article.

## Description

### Technical Field

The invention relates to a method of post-treatment of a 3D-printed silicone elastomer article. More specifically, the invention relates to a method consisting in post-treating a 3D-printed silicone elastomer article, in particular its surface, with a specific post-treatment composition. The invention further relates to said specific post-treatment composition, to the post-treated 3D-printed silicone elastomer article, and to an additive manufacturing process containing such post-treatment method.

### Background of the invention

Three-dimensional (3D) printing is a process in which material can be deposited, joined or solidified under computer control to create a three-dimensional object with material being added together layer by layer. Additive manufacturing techniques cover different technologies in which objects are obtained layer by layer via specific techniques such as extrusion, sintering, melting, photopolymerization, jetting, lamination and deposition.

Silicone has been used for a variety of medical applications mainly owing to its biocompatibility and thermal stability. Medical-grade silicones are used in anatomic models, mannequins, prosthetics, pharmaceuticals, lubricating injection devices etc. Producing silicone structures by 3D printing is a promising method with high design flexibility.

However, the object printed by such 3D printing process, although faithful to the geometry of the original electronic file used for printing, may also reflect some surface issues. Indeed, 3D printing technology is based on a layer-by-layer printing process. Due to the layer-by-layer printing or deposition in the 3D printing process, the surface or the contour of the 3D-printed object usually has a wrinkled or wave-like appearance, which can be referred as a step effect or sometimes as aliasing. The step effect is related to several issues:
- The step effect may lead to aesthetic issues, which can be unacceptable when the 3D-printed article is intended to be used for instance as a prosthesis. It is highly desirable that the surface of the 3D-printed article shows a skin or soft touch feel, similar to human skin.
- Additionally, the mechanical and/or physical properties of the 3D-printed article may be affected by the excessive surface roughness, depending on the final application. For example, when the 3D-printed article is a 3D-printed silicone tracheal patch used to repair tracheal defects, a high roughness can trigger an excessive inflammatory reaction in the patient with tracheostomy.

In order to remove the step effect, different methods have been proposed. For metallic materials, polishing, anodic oxidation, sand blasting, powder coating and electroplating are usually used to obtain a smooth surface and contour. As for the plastic or polymeric objects, some mechanical treatments like sand blasting or polishing may be used in the surface finishing to remove the step effects. However, it appears that none of these standard methods seem suitable when 3D-printed articles are silicone elastomers.

The US patent applications US 2020/0108548 A1, US 2020/0316850 A1 and US 2020/0238601 A1 disclose the possibility of carrying out a post-treatment on 3D-printed silicone articles in order to improve the surface quality of said silicone articles. However, these documents do not disclose any details about the post-treatment steps, nor any examples.

The international patent application WO 2022/141374 A1 discloses a method of post-treatment of the surface of an additive manufactured object, comprising the steps of coating at least part of the surface with a curable silicone composition and then curing the coating at room temperature or by heat or UV radiation, characterized in that the curable silicone composition has the viscosity in a range from 300 mPa.s to 500 000 mPa.s. According to this document, this post-treatment process may greatly or totally remove the step effect, without impairing the adhesion and mechanical properties of the surface. However, this document is silent about the improvement of the skin-touch feeling of the surface.

It is an object of the present invention to provide a method of post-treatment of a 3D-printed silicone article for reducing the step effect. It is also an object of the present invention to provide a method for additive manufacturing of a 3D silicone elastomer article having a surface with improved skin-touch feeling.

### Summary of the invention

All these objectives, among others, are achieved by the present invention, which relates to a method of post-treatment of a 3D-printed silicone article, said method comprising coating the 3D-printed silicone article with a crosslinkable silicone composition comprising a microfiller.

The invention further relates to a crosslinkable silicone composition comprising a microfiller, and its use as post-treatment composition for the post-treatment of a 3D printed silicone article.

The present invention further relates to a method for additive manufacturing of a 3D silicone elastomer article comprising the steps of:
a) additive manufacturing a silicone article, and
b) coating the 3D-printed silicone article with a crosslinkable silicone composition comprising a microfiller.

### Detailed description of the invention

In the absence of other indication, the viscosities of the silicone compositions and their individual constituents described herein correspond to a "Newtonian" dynamic viscosity magnitude at indicated temperature, i.e. the dynamic viscosity which is measured, in a manner that is known per se, with a Haake rheometer at a shear rate gradient that is low enough for the measured viscosity to be independent of the shear rate gradient.

Unless otherwise specified, the contents in % or ppm are by weight.

As used herein, the term "silicone elastomer" includes the crosslinked product of any crosslinkable silicone composition. The terms "silicone elastomer" and "silicone rubber" may be used interchangeably.

As used herein, the terms "crosslinked" and "cured" may be used interchangeably and refer to the reaction that occurs when the components of the crosslinkable silicone composition are combined and allowed to react, resulting in the crosslinked silicone elastomer.

According to ISO/ASTM Standard 52900 ("Additive manufacturing - General principles - Terminology", 2017), "additive manufacturing (AM)" is defined as a process of joining materials to make parts from 3D model data, usually layer upon layer, as opposed to subtractive manufacturing and formative manufacturing methodologies. Synonyms associated with AM include additive fabrication, additive processes, additive techniques, additive layer manufacturing, layer manufacturing, solid freeform fabrication, and freeform fabrication. Besides, "3D printing" is defined as the fabrication of objects through the deposition of a material using a print head, nozzle, or another printer technology. It is a term often used in a non-technical context synonymously with additive manufacturing. As used herein, "3D printing" is generally interchangeable with "additive manufacturing" and vice versa. "3D printer" is defined as a machine used for 3D printing.

The present invention relates to a method of post-treatment of a 3D-printed silicone article, said method comprising the coating the 3D-printed silicone article with a crosslinkable silicone composition comprising a microfiller.

The crosslinkable silicone composition according to the present invention comprises a microfiller. A microfiller according to the present invention is a filler material having a mean particle size between 1 µm and 1000 µm. As used herein, "mean particle size", also referred to as "D50", is the value of the particle diameter at 50% in the cumulative volume distribution. Particle diameter can typically be measured by static laser scattering. The microfiller can advantageously be selected from the group of powders comprising spherical (or substantially spherical) particles. The spherical particles refer to particles of a spherical shape having one or more nearly spherical diameter across the centroid or geometric center and may be spherical particles having uneven surface. In particular, the spherical particles have a ratio of shortest diameter to longest diameter from 0.2 to 1, preferably 0.3 to 1 or such as 0.4 to 0.5, or 0.6 to 0.9, or 0.7 to 0.9.

Microfiller can be selected from the group consisting of low-density hollow fillers, powders of (co)polyamides, silica or glass microfillers and spherical silicone resin particles.

According to a first preferred embodiment, the crosslinkable silicone composition according to the present invention comprises a low-density hollow filler. Said low-density hollow filler can preferably have a true density below 1 g/cm³ (gram per cubic centimeter), more preferably below 0.8 g/cm³, even more preferably below 0.5 g/cm³. According to an embodiment, the low-density hollow filler can have a true density comprised between 0.01 g/cm³ and 1 g/cm³.

In this document, the expression "true density" is the value obtained by dividing the mass of a sample of fillers by the true volume of that mass of fillers as measured by a gas pycnometer. The "true volume" is the aggregate total volume of the fillers, not the bulk volume. True density (also sometimes referred to as "specific density" or "effective density") is thus the particle density as measured by liquid displacement, whereas bulk density is the weight as measured in a container, and includes the interstitial air.

According to a preferred embodiment, the low-density hollow filler according to the invention is hollow microspheres, preferably selected from mineral hollow microsphere and organic hollow microsphere.

As examples of suitable mineral hollow microspheres, it can be cited hollow glass microspheres or hollow ceramic microspheres.

Hollow glass microspheres, also sometimes referred to as "hollow glass beads" or "hollow glass bubbles", are small hollow spheres of hardened silica (glass) that can vary in size and density depending on the grade. They have a shell that is thick enough to maintain structural rigidity. Due to their hollow nature, they are very lightweight, with a density that varies with size and wall thickness. In bulk they appear as a white powder. The main differences between grades are in their size, strength and density, with the strength of the microspheres being expressed in terms of their average isostatic crushing strength.

According to an embodiment, hollow glass beads are hollow borosilicate glass microspheres.

According to an embodiment, the hollow glass microspheres have a true density ranging from 0.10 g/cm³ to 0.75 g/cm³.

According to a preferred embodiment, hollow glass microspheres are selected from:
1) 3M^{™} Glass Bubbles Floated Series (A16/500, G18, A20/1000, H20/1000, D32/4500 and H50/10,000EPX glass bubbles products) and 3M^{™} Glass Bubbles K, S, iM and XLD Series (such as but not limited to K1, K11, K15, S15, S22, K20, K20HS, K25, S32, S32LD, S35, XLD3000, S28HS, S35, K37, S38, S38HS, S38XHS, S32HS, K46, K42HS, S42XHS, S60, S60HS, iM16K, iM30K glass bubbles products) sold by 3M Company. Said glass bubbles exhibit various crush strengths ranging from 1.72 MPa (250 psi) to 186.15 MPa (27,000 psi) at which ten percent by volume of the first plurality of glass bubbles collapses. Other glass bubbles sold by 3M such as 3M^{™} Glass Bubbles - HGS Series and 3M^{™} Glass Bubbles with Surface Treatment could also be used according to the invention; and
2) Hollow glass microspheres sold under the tradename SPHERICEL^{®} (products such as: 110P8, 60P18, 34P30, and 25P45) or under the tradename Q-Cel^{®} Lightweight (products such as: 6014, 6019, 7019, 6019S, 5020, 5020FPS, 7023, 7028, 2058, 6036, 7037, 7040S, 6042S, 6048 and 5070S), sold by Potters Industries Inc.

Hollow ceramic microspheres also referred to as cenospheres are lightweight, inert, hollow sphere filled with inert air or gas, typically produced as a byproduct of coal combustion at thermal power plants. They are made largely of silica and alumina. The color of cenospheres varies from gray to almost white and their true density is about 0.4 g/cm³ to 0.8 g/cm³. It flows like a liquid, with the appearance of a powder. Suitable cenospheres are not surface-treated or are surface-treated with a silane-based coupling agent such as one or more of 3-aminopropyltriethoxysilane, gammaglycidoxypropyltrimethoxysilane, gamma- (methacryloyloxy) propyltrimethoxysilane, 3-aminopropyltrimethoxysilane, 4-aminopropylmethyldimethoxysilane or 3-aminopropylmethyldiethoxysilane.

Commercially available examples of hollow ceramic microspheres are Z-Light^{™} Spheres Microspheres commercialized by 3M^{™} (products such as: 3M^{™} Z-Light^{™} Spheres G-3125, G-3150 and G-3500).

As examples of suitable organic hollow microspheres, it can be any organic hollow microspheres consisting in a thermoplastic sphere encapsulating a gas. The thermoplastic polymer can be any organic polymers, for instance polyethylene (PE), polyurethane (PU), polysulphide (PS), or polymethyl methacrylate (PMMA). Commercially available examples include Expancel^{®} microspheres from Akzo Nobel (now Nouryon).

The low-density hollow filler according to the present invention can preferably have a mean particle size between 1 µm and 1000 µm. Typically, the hollow glass microspheres used in the compositions are essentially homogeneous and essentially uniform in sphericity and have a mean particle size of between about 5 µm and 500 µm, such as from about 10 µm to 70 µm. Typically, the organic hollow microspheres used in the compositions are essentially homogeneous and essentially uniform in sphericity and have a mean particle size of between about 10 µm and 1000 µm, such as from about 10 µm to 70 µm. In the composition according to the present invention, it is preferred to select a low-density hollow filler having a mean particle size below 600 µm, more preferably below 400 µm, more preferably below 200 µm, even more preferably below 100 µm.

According to a second embodiment, the crosslinkable silicone composition according to the present invention comprises powders of (co)polyamides, preferably the (co)polyamides 6, 12 and 6/12.

Powders of (co)polyamides can comprise particles substantially rounded in shape and with mean particle size comprised between 1 µm and 200 µm, preferably between 5 µm and 100 µm, and more preferably between 10 µm and 50 µm. Powders of (co)polyamide can show a density of between 1 g/cm³ and 1.2 g/cm³ .

Commercially available examples of (co)polyamide powders are in particular Orgasol^{®} powder commercialized by Arkema (products such as: Orgasol^{®} 1002 D NAT 1, Orgasol^{®} 1002 ES5 NAT 1, Orgasol^{®} 2001 EXD NAT 1, Orgasol^{®} 2001 UD NAT 1, Orgasol^{®} 2001 UD NAT 2, Orgasol^{®} 2002 D NAT 1, Orgasol^{®} 2002 ES3 NAT 3, Orgasol^{®} 2002 ES4 NAT 3, Orgasol^{®} 2002 ES5 NAT 3, Orgasol^{®} 2002 ES6 NAT 3, Orgasol^{®} 2002 EXD NAT 1, Orgasol^{®} 3501 EXD NAT 1, Orgasol^{®} 3502 D NAT 1).

According to a third embodiment, the crosslinkable silicone composition according to the present invention comprises a silica or glass microfiller.

Silica microfiller can be selected from the group of silica powders having a mean particle size above 1 µm. These silicas can advantageously be colloidal silicas, combustion silicas, precipitation silicas, or their mixtures. Silica microfillers can be untreated or treated, in particular the surface of the silica particles may be rendered hydrophobic. Rendering the filler particles hydrophobic may be done either prior to or after dispersing the precipitated silica particles in the polysiloxane component. This can be affected by pre-treatment of the silica particles with the hydrophobing agents like fatty acids, reactive silanes, wax or reactive siloxanes. Examples include but are not limited to stearic acid, dimethyldichlorosilane, trimethylchlorosilane, hexamethyldisilazane, hydroxyl end blocked or methyl end blocked polydimethylsiloxanes, siloxane resins or mixtures of two or more of these. The precipitated silica particles which have already been treated hydrophobic are commercially available in the market. Most preferred hydrophobing agent is hexamethyldisilazane or polyethene wax.

Commercially available examples of silica microfillers are Acematt^{®} silica and Spherilex^{®} silica, both by Evonik (products such as: Acematt^{®} OK 607, Acematt^{®} 3300, Acematt^{®} 3600, Acematt^{®} TS 100, Spherilex^{®} 30 A/B, Spherilex^{®} 60 A/B, Spherilex^{®} DP-0111, Spherilex^{®} DP-0112, and Spherilex^{®} DP-0115), ENOVA^{®} aerogel (silica aerogel particles) from Cabot (products such as ENOVA^{®} MT1100 and MT1200), and SYLOID^{®} silicas by Grace (products such as SYLOID^{®} 7000, SYLOID^{®} C906, and SYLOID^{®} ED30).

Glass microfillers can be selected from the group of glass beads having a mean particle size above 1 µm. Similarly to silica microfillers, glass beads can be untreated or treated. Commercially available examples of glass microfillers are Spheriglass^{®} solid glass spheres by Potters (A-GLASS series or E-GLASS series) and Microperl^{®} solid glass beads by Sovitec.

According to a fourth embodiment, the crosslinkable silicone composition according to the present invention comprises spherical silicone resin particles. Spherical silicone resin particles can be spherical polysilsesquioxane (preferably polymethylsilsesquioxane) particles, and exhibit a substantially spherical shape.

"Polysilsesquioxane particles" means that the particles comprise at least 50 mol.% T units based on the sum of M, D, T and Q units in the molecule. Preferably, the polysilsesquioxane particles comprise 60 mol.% or more, 70 mol.% or more, 80 mol.% or more, 90 mol.% or more and can be 100 mol.% T units based on the sum of M, D, T and Q units. "Polymethylsilsesquioxane" means polysilsesquioxane wherein the substituting group of the T unit is essentially methyl.

Commercially available examples of spherical silicone resin particles are Mirasil^{™} Micropearl 40 by Elkem Silicones, silicone resin powders available from Shin-Etsu Chemical Co., Ltd. (products such as KMP-706, KMP-701 or X-52-1621), and Tospearl^{™} silicone beads provided by Momentive.

Any microfiller according to the present invention can be added as such directly into the crosslinkable silicone composition during the preparation of the mixture or added into the mixture in form of dispersion of particles in the diluent.

The amount of microfiller in the crosslinkable silicone composition according to the invention can preferably in the range from 0.1 wt.% to 50 wt.%, preferably 1 wt.% to 20 wt.%, more preferably 2 wt.% to 15 wt.%, and even more preferably 5 wt.% to 10 wt.%, with respect to the total weight of the crosslinkable silicone composition.

The microfiller as described above is comprised in a crosslinkable silicone composition. According to a preferred embodiment, the crosslinkable silicone composition is a polyaddition-crosslinkable silicone composition, i.e. is able to crosslink through polyaddition reactions.

The crosslinkable silicone composition can comprise:
(A) at least one organopolysiloxane compound **A** comprising, per molecule, at least two alkenyl radicals bonded to silicon atoms,
(B) at least one organohydrogenopolysiloxane compound **B** comprising, per molecule, at least two hydrogen atoms bonded to an identical or different silicon atom,
(C) at least one polyaddition catalyst **C,** preferably a polyaddition catalyst consisting of at least one metal or compound from the platinum group,
(D) at least one microfiller **D** as described above.

Optionally, the crosslinkable silicone composition can comprise at least one crosslinking inhibitor **E.**

Optionally, the crosslinkable silicone composition can further comprise at least one reinforcing filler **F.**

Optionally, the crosslinkable silicone composition can further comprise at least one solvent **G.**

This silicone composition is crosslinkable through polyaddition reactions between organopolysiloxanes bearing at least two unsaturated bonds and organopolysiloxanes bearing at least two hydrogenosilyl units.

Preferably, the organopolysiloxane A exhibits, per molecule, at least two C₂₋₁₂ alkenyl groups bonded to the silicon. It can consists of at least two siloxyl units of following formula: YₐR¹_{b}SiO(_{4-a-b)/2} in which:
- Y is a C₂₋₁₂ alkenyl, preferably a C₂₋₆ alkenyl, more preferably vinyl,
- R¹ is a monovalent hydrocarbon group having from 1 to 12 carbon atoms, optionally substituted by one or several halogen atoms, preferably selected from the alkyl groups having from 1 to 8 carbon atoms, such as the methyl, ethyl or propyl groups, cycloalkyl groups having from 3 to 8 carbon atoms and aryl groups having from 6 to 12 carbon atoms,
- a = 1 or 2, b = 0, 1 or 2 and the sum a + b = 2 or 3,
and optionally units of following formula: R¹_{c}SiO_{(4-c)/2}
in which R¹ has the same meaning as above and c = 0, 1, 2 or 3.

In the present disclosure, reference is made to the following nomenclature in order to represent the siloxyl units:
M: R¹₃SiO_{1/2} siloxyl unit,
M^{Vi}: siloxyl unit selected from the group consisting of the YR¹₂SiO_{1/2} and Y₂R¹SiO_{1/2};
D: R¹₂SiO_{2/2} siloxyl unit,
D^{Vi}: siloxyl unit selected from the group consisting of Y₂SiO_{2/2} or YR¹SiO_{2/2} siloxyl units,
T: siloxyl unit of formula R¹SiO_{3/2},
Q: siloxyl unit of formula SiO_{4/2},
wherein the symbols Y and R¹ are as described above

As examples of terminal "M" units, mention may be made of the trimethylsiloxy or dimethylphenyl-siloxy groups.

As examples of terminal "M^{Vi}" units, mention may be made of the dimethylvinylsiloxy or dimethyl-hexenylsiloxy groups.

As examples of "D" units, mention may be made of the dimethylsiloxy and methylphenylsiloxy groups.

As examples of "D^{Vi}" units, mention may be made of the methylvinylsiloxy, methylbutenylsiloxy, methylhexenylsiloxy, methyldecenylsiloxy or methyldecadienylsiloxy groups.

As examples of "T" units, mention may be made of the methylsiloxy group.

The organopolysiloxane **A** can be a linear organopolysiloxane, a cyclic organopolysiloxane or a resin (i.e. branched organopolysiloxane). The crosslinkable silicone composition according to the present invention can comprise a mixture of different organopolysiloxanes **A.**

According to one embodiment, the organopolysiloxane **A** can be a linear organopolysiloxane **A1**. Linear organopolysiloxanes exhibit a linear structure essentially formed of D or D^{Vi} siloxyl units, and of terminal M or M^{Vi} siloxyl units.

Examples of linear organopolysiloxanes which can be organopolysiloxane **A** according to the invention are:
- a poly(dimethylsiloxane) comprising dimethylvinyl-silyl terminations;
- a poly(dimethylsiloxane-co-methylpheylsiloxane) comprising dimethylvinylsilyl terminations;
- a poly(dimethylsiloxane-co-methylvinylsiloxane) comprising dimethylvinylsilyl terminations;
- a poly(dimethylsiloxane-co-methylvinylsiloxane) comprising trimethylsilyl terminations; and
- a cyclic poly(methylvinylsiloxane).

According to a preferred embodiment, the organopolysiloxane **A1** contains terminal dimethylvinylsilyl units, and even more preferably the organopolysiloxane **A1** is a poly(dimethylsiloxane) comprising terminal dimethylvinylsilyl groups. The number of dimethylsiloxane units can be comprised between 5 to 1000, and preferably from 100 to 600.

Preferably, the organopolysiloxane **A1** can have a dynamic viscosity at 25°C of between 100 mPa.s and 120,000 mPa.s, preferably between 100 mPa.s and 80,000 mPa.s, more preferentially between 1,000 mPa.s and 50,000 mPa.s, and even more preferably between 5,000 mPa.s and 20,000 mPa.s. Said organopolysiloxane **A1** can preferably be referred to as an organopolysiloxane oil.

Preferably, the organopolysiloxane **A1** has a content by weight of alkenyl unit of between 0.001% and 30%, preferably between 0.01% and 10%, preferably between 0.02% and 5%.

According to another embodiment, the organopolysiloxane **A** can be a branched organopolysiloxane **A2** (i.e. a resin) comprising alkenyl units. It is preferably selected from the group consisting of the silicone resins of following formulas:
- M^{Vi}Q, where the alkenyl groups bonded to silicon atoms are carried by the M groups,
- MM^{Vi}Q, where the alkenyl groups bonded to silicon atoms are carried by a part of the M units,
- MD^{Vi}Q, where the alkenyl groups bonded to silicon atoms are carried by the D groups,
- MDD^{Vi}Q, where the alkenyl groups bonded to silicon atoms are carried by a part of the D groups,
- MM^{Vi}TQ, where the alkenyl groups bonded to silicon atoms are carried by a part of the M units,
- MM^{Vi}DD^{Vi}Q, where the hydrogen atoms bonded to silicon atoms are carried by a part of the M and D units,
- and their mixtures.

According to a preferred embodiment, the crosslinkable silicone composition according to the present invention comprises a mixture of at least one linear organopolysiloxanes **A1** as defined above and of at least one branched organopolysiloxane (i.e. resin) **A2** as defined above.

The total amount of organopolysiloxane compound **A** comprising, per molecule, at least two alkenyl radicals bonded to silicon atoms, in the crosslinkable silicone composition according to the invention can preferably in the range from 40 wt.% to 95 wt.%, preferably 50 wt.% to 90 wt.%, with respect to the total weight of the crosslinkable silicone composition.

The crosslinkable silicone composition according to the present invention can further comprise at least one organosilicon compound **B** having at least two, and preferably at least three, hydrogen atoms bonded to silicon, per molecule. The organosilicon compound **B** is preferably an organohydrogenpolysiloxane compound comprising, per molecule, at least two and preferably at least three hydrosilyl functional groups (or Si-H units).

The organosilicon compound **B** can advantageously be an organopolysiloxane comprising at least two, preferably at least three, siloxyl units of following formula: H_{d}R²ₑSiO_{(4-d-e)/2}
in which:
- the R² radicals, which are identical or different, represent a monovalent radical having from 1 to 12 carbon atoms,
- d = 1 or 2, e = 0, 1 or 2 and d + e = 1, 2 or 3;
and optionally other units of following formula: R²_{f}SiO_{(4-f)/2}
in which R² has the same meaning as above and f = 0, 1, 2 or 3.

It is understood that, in the formulas above, if several R² groups are present, they can be identical to or different from one another. Preferentially, R² can represent a monovalent radical selected from the group consisting of alkyl groups having from 1 to 8 carbon atoms, optionally substituted by at least one halogen atom, such as chlorine or fluorine, cycloalkyl groups having from 3 to 8 carbon atoms and aryl groups having from 6 to 12 carbon atoms. R² can advantageously be selected from the group consisting of methyl, ethyl, propyl, 3,3,3-trifluoropropyl, xylyl, tolyl and phenyl, and most preferentially R² is methyl.

The symbol d is preferentially equal to 1.

In the present disclosure, reference is made to the following nomenclature in order to represent the siloxyl units:
M: R²₃SiO_{1/2} siloxyl unit,
M': R²₂HSiO_{1/2} siloxyl unit,
D: R²₂SiO_{2/2} siloxyl unit,
D': R²HSiO_{2/2} siloxyl unit,
T: R²SiO_{3/2}, siloxyl unit, and
Q: SiO_{4/2} siloxyl unit,
wherein R² has the same meaning as above.

The organosilicon compound **B** can exhibit a linear, branched or cyclic structure. The degree of polymerization is preferably greater than or equal to 2. Generally, it is less than 5000.

Preferably, the viscosity of the organosilicon compound **B** is between 1 mPa.s and 5,000 mPa.s, more preferentially between 1 mPa.s and 2,000 mPa.s and more preferentially still between 5 mPa.s and 1,000 mPa.s.

Examples of organohydrogenpolysiloxanes which can be organosilicon compound **B** according to the invention comprising at least two hydrogen atoms bonded to a silicon atom are:
- a poly(dimethylsiloxane) comprising hydrodimethyl-silyl terminations;
- a poly(dimethylsiloxane-co-methylhydrosiloxane) comprising trimethylsilyl terminations;
- a poly(dimethylsiloxane-co-methylhydrosiloxane) comprising hydrodimethylsilyl terminations;
- a poly(methylhydrosiloxane) comprising trimethyl-silyl terminations; and
- a cyclic poly(methylhydrosiloxane).

When the organosilicon compound **B** exhibits a branched structure, it is preferably selected from the group consisting of the silicone resins of following formulas:
- M'Q, where the hydrogen atoms bonded to silicon atoms are carried by the M groups,
- MM'Q, where the hydrogen atoms bonded to silicon atoms are carried by a part of the M units,
- MD'Q, where the hydrogen atoms bonded to silicon atoms are carried by the D groups,
- MDD'Q, where the hydrogen atoms bonded to silicon atoms are carried by a part of the D groups,
- MM'TQ, where the hydrogen atoms bonded to silicon atoms are carried by a part of the M units,
- MM'DD'Q, where the hydrogen atoms bonded to silicon atoms are carried by a part of the M and D units,
- and their mixtures.

Preferably, the organosilicon compound **B** has a content by weight of hydrosilyl Si-H functional groups of between 0.2% and 91%, more preferentially between 3% and 80% and more preferentially still between 15% and 70%.

Advantageously, the molar ratio of the hydrosilyl SiH functional groups of the organosilicon compound **B** to the alkene functional groups of the compound **A** is between 5 and 100, preferably between 10 and 90, more preferentially between 15 and 65 and more preferentially still between 20 and 55.

According to one preferred embodiment, the crosslinkable silicone composition according to the present invention comprises a mixture of at least one organosilicon compound **B1** having at least three hydrogen atoms bonded to silicon per molecule and at least one organosilicon compound **B2** having two hydrogen atoms bonded to silicon per molecule. Said organosilicon compound **B2** contains preferably terminal dimethylhydrogensilyl units, and even more preferably the organosilicon compound **B2** is a poly(dimethylsiloxane) comprising terminal dimethylhydrogensilyl groups. The number of dimethylsiloxane units can be comprised between 1 to 200, preferably between 1 and 150, and more preferably between 3 and 120. Such organosilicon compound **B2** can be described as "chain extender" since it has the presumed effect of increasing the mesh size of the network when it is crosslinked. Besides, such organosilicon compound **B1** having three hydrogen atoms bonded to silicon per molecule or more can be described as "crosslinker". Preferably the organosilicon compound **B1** is a poly(dimethylsiloxane-co-methylhydrosiloxane) comprising trimethylsilyl terminations and/or hydrodimethylsilyl terminations.

The total amount of the organosilicon compound **B** having at least two hydrogen atoms bonded to silicon, per molecule, in the crosslinkable silicone composition according to the invention can preferably in the range from 1 wt.% to 20 wt.%, preferably 3 wt.% to 15 wt.%, with respect to the total weight of the crosslinkable silicone composition.

The hydrosilylation catalyst **C** can in particular be selected from platinum and rhodium compounds but also from silicon compounds, such as those described in the patent applications WO 2015/004396 and WO 2015/004397, germanium compounds, such as those described in the patent application WO 2016/075414, or nickel, cobalt or iron complexes, such as those described in the patent applications WO 2016/071651, WO 2016/071652 and WO 2016/071654. The catalyst C is preferably a compound derived from at least one metal belonging to the platinum group. These catalysts are well known. It is possible in particular to use complexes of platinum and of an organic product described in the patents US 3,159,601, US 3,159,602 and US 3,220,972 and the European patents EP 0057459, EP 0188978 and EP 0190530, or the complexes of platinum and of vinylated organosiloxanes described in the patents US 3,419,593, US 3,715,334, US 3,377,432 and US 3,814,730. Alternatively, a hydrosilylation photocatalyst can be used. Said catalyst can be activated by irradiation, preferably by UV radiation. Examples of suitable platinum-based photocatalysts include: bis(acetylacetonate)platinum, trimethyl(acetylacetonate)platinum complex, trimethyl(2,4-pentanedione)platinum complex, trimethyl(3,5-heptanedione)platinum complex, trimethyl(methyl acetoacetate)platinum complex, bis(2,4-pentanedione)platinum complex , bis(2,4-hexanedione)platinum complex, bis(2,4-heptanedione)platinum complex, bis(3,5-heptanedione)platinum complex and bis(1-phenyl-1,3-butanedione)platinum complex.

Preferentially, the catalyst **C** is a compound derived from platinum. Preferentially, the catalyst **C** is a Karstedt platinum catalyst.

The amount of the catalyst **C** in the crosslinkable silicone composition according to the invention can preferably in the range from 2 ppm to 400 ppm, preferably from 5 ppm to 200 ppm, calculated as weight of platinum metal, with respect to the total weight of the crosslinkable silicone composition.

Optionally, the crosslinkable silicone composition can comprise at least one crosslinking inhibitor **E.**

Crosslinking inhibitors, which are also known as cure rate controller, are designed to slow the curing of the compounded silicone if needed. Crosslinking inhibitors are well known in the art and examples of such materials can be found in U.S. Patents. US 3,923,705 refers to the use of vinyl contained cyclic siloxanes. US 3,445,420 describes the use of acetylenic alcohols. US 3,188,299 shows the effectiveness of heterocyclic amines. US 4,256,870 describes alkyl maleates used to control cure. Olefinic siloxanes can also be used as described in US 3,989,667. Polydiorganosiloxanes containing vinyl radicals have also been used and this art can be seen in US 3,498,945, US 4,256,870, and US 4,347, 346. Preferred inhibitors for this composition are 1,3,5,7-tetramethyl-1,3,5,7-tetravinyl-cyclotetrasiloxane, 3-methyl-1-butyn-3-ol, and 1-ethynyl-1-cyclohexanol (ECH), with the most preferred being the 1-ethynyl-1-cyclohexanol.

The content of crosslinking inhibitor **E** can vary depending on the desired curing rate. To obtain a longer "gel time" (also known as working time or "pot life"), the quantity of the crosslinking inhibitor **E** is adjusted to reach the desired "gel time". The concentration of the crosslinking inhibitor **E** in the present silicone composition is sufficient to retard curing of the composition at ambient temperature without preventing or excessively prolonging cure at elevated temperatures. This concentration will vary widely depending on the particular inhibitor used, the nature and concentration of the hydrosilylation catalyst, and the nature of the organohydrogenpolysiloxane. Inhibitor concentrations as low as one mole of inhibitor per mole of platinum group metal will in some instances yield a satisfactory storage stability and cure rate. In other instances, inhibitor concentrations of up to 500 or more moles of inhibitor per mole of platinum group metal may be required. The optimum concentration for a particular inhibitor in a given silicone composition can be readily determined by the person skilled in the art.

Optionally, the crosslinkable silicone composition can further comprise at least one thixotropic agent **F.** A thixotropic agent is a rheological agent which serves to adjust the shear-thinning and thixotropic characteristics. In the present invention, it was discovered that the addition of a thixotropic agent might advantageously improve the stability of the crosslinkable silicone composition during storage. The phase separation effect is reduced or can even disappear.

The optional thixotropic agent **F** can preferably be a reinforcing silica filler. It can in particular be selected from colloidal silicas, combustion and precipitated silica powders, or mixtures thereof. These powders have an average particle size that is less than 100 nm (nanometers) and a BET specific surface area greater than 30 m²/g, preferably greater than 50 m²/g, more preferably from 100 m²/g to 400 m²/g.

Reinforcing fillers of this kind are very well-known materials within the field of the silicone rubbers. Silica fillers may have hydrophilic character or may have been hydrophobized by known processes.

The silica reinforcing fillers can be non-treated or can be subjected to an overall surface treatment, preferably hydrophobic treatment. Surface treatment can be carried out preliminary to the addition of silica to the silicone composition, or alternatively a surface treatment agent may be added during mixing of the with the organopolysiloxane compounds, so that the silica is treated in-situ.

Alternatively, the optional thixotropic agent **F** can be an organic or organosilicon compound containing polar groups. Preferably, the thixotropic agent **F** can be selected from the group consisting of: an organic or organosilicon compound having at least one epoxy group, an organic or organopolysiloxane compound having at least one (poly)ether group, an organic compound having at least (poly)ester group, an organopolysiloxane having at least one aryl group, or with tetramethylpiperidine group known as "HALS" (Hindered Amine Light Stabilizer) and any combination thereof.

For example, there may be mentioned the polyorganosiloxane compounds containing, per molecule, at least one siloxyl unit substituted with at least one group having one or more sterically hindered piperidinyl functional groups, provided by the company Elkem Silicones under the name COMFORTSIL^{®} FLD 21645, COMFORTSIL^{®} FLD 21650, or COMFORTSIL^{®} FLD HYDROSOFT.

According to an embodiment, the thixotropic agent **F** is an organopolysiloxane having at least one aryl group, more preferably an aryl-group containing organopolysiloxane gums. For example, the compounds disclosed in the international patent application WO 2021/211752, especially a diphenylsiloxane-dimethylsiloxane-vinylmethylsiloxane copolymer gum or a vinyldimethyl terminated diphenylsiloxane-dimethylsiloxane copolymer gum, can be suitable.

According to an embodiment, the thixotropic agent **F** is an organopolysiloxane-polyoxyalkylene copolymer, also known as polydiorganosiloxane-polyether copolymers or polyalkylene oxide modified polyorganosiloxanes, are organopolysiloxanes containing siloxyl units which carry alkylene oxide chain sequences. Preferably, organopolysiloxane-polyoxyalkylene copolymer are organopolysiloxanes containing siloxyl units which carry ethylene oxide chain sequences and/or propylene oxide chain sequences. An example of organopolysiloxane-polyoxyalkylene copolymer that can be used in the method of the invention corresponds to the following formula:

R^{a}₃SiO[R^{a}₂SiO]ₜ[R^{a}Si(R^{b}-(OCH₂CH₂)ₓ(OCH(CH₃)CH₂)_{y}-OH)O]ᵣSiR^{a}₃

wherein:
- each R^{a} is independently selected from alkyl groups containing from 1 to 8 carbon atoms and preferably R^{a} is a methyl group,
- each R^{b} is a divalent hydrocarbon group having from 2 to 6 carbon atoms or a direct bond, and preferably R^{b} is a propyl group,
- x and y are independently integers comprised from 1 to 40, preferably from 5 and 30, and most preferably from 10 to 30,
- t is comprised from 1 to 200, preferably from 25 to 150, and
- r is comprised from 2 to 25, preferably from 3 to 15.

Advantageously, in an embodiment the organopolysiloxane-polyoxyalkylene copolymer is: Me₃SiO[Me₂SiO]₇₅ [MeSi((CH₂)₃-(OCH₂CH₂)₂₂(OCH(CH₃)CH₂)₂₂-OH)O]₇SiMe₃.

In another embodiment, the organopolysiloxane-polyoxyalkylene copolymer is a branched organopolysiloxane-polyoxyalkylene copolymer comprising at least one T and/or one Q siloxy unit with Q corresponding to the siloxy unit SiO_{2/2} and T corresponding to the siloxy unit R¹SiO_{3/2} where R¹ is independently selected from hydrocarbon-based group containing from 1 to 30 carbon atoms, preferably chosen from the group formed by alkyl groups containing from 1 to 8 carbon atoms, alkenyl groups containing from 2 to 6 carbon atoms and aryl groups containing between 6 and 12 carbon atoms

In another embodiment, the organopolysiloxane-polyoxyalkylene copolymer can further comprise other functional groups chosen from the group consisting of: alkenyl groups having from 2 to 6 carbon atoms, hydroxide, hydrogen, (meth)acrylate groups, amino groups and hydrolysable groups as alkoxy, enoxy, acetoxy or oxime groups.

According to the invention, thixotropic agents **F** can be used alone or in combination.

The total amount of the thixotropic agent **F** in the silicone composition is in the range from 0.2 wt.% to 40 wt.%, preferably 0.2 wt.% to 20 wt.%, and more preferably 0.2 wt.% to 15 wt.% by weight of the total composition.

When the thixotropic agent **F** is a reinforcing silica filler, the amount of the thixotropic agent **F** in the silicone composition can preferably in the range from 0.5 wt.% to 40 wt.%, preferably 1 wt.% to 20 wt.%, and more preferably 2 wt.% to 5 wt.%, with respect to the total weight of the silicone composition.

When the thixotropic agent **F** is an organic or organosilicon compound containing polar groups, the amount of the thixotropic agent **F** in the silicone compositions can be at least 0.2 wt.%, preferably at least 0.25 wt.%, most preferably in the range from 0.25 wt.% to 4 wt.%, and even most preferably from 0.25 wt.% to 3 wt.%, with respect to the total weight of the silicone composition.

Optionally, the crosslinkable silicone composition can further comprise at least one solvent **G.** The function of a solvent is here to dissolve said crosslinkable silicone composition prior to coating on the 3D-printed silicone object. The solvent **G** can be preferably selected from the group consisting of non-reactive polyorganosiloxane like decamethylcyclopentasiloxane (D5), octamethylcyclotetrasiloxane (D4), or a polydimethylsiloxane of low viscosity (typically below 50 mPa.s), and organic solvent like DMF, chlorobenzene, xylene, toluene, acetonitrile, ethanol, THF, chloroform, ethyl acetate, cyclohexane, butanone, acetone, and petroleum ether.

When a solvent **G** is present in the silicone composition, the amount of solvent **G** can preferably in the range from 1 wt.% to 80 wt.%, preferably 10 wt.% to 60 wt.%, with respect to the total weight of the silicone composition.

However, according to a preferred embodiment, the crosslinkable silicone composition according to the invention comprises less than 30 wt.%, more preferably less than 10 wt.%, and even more preferably less than 1 wt.% of organic solvent. The crosslinkable silicone composition according to the invention can be free of, or substantially free of, organic solvent. Advantageously, a low content or the absence of organic solvent makes the crosslinkable silicone composition more environmental-friendly and less vulnerable to the bacteria. Therefore, the post-treatment method according to the invention and the 3D-printed article treated thereby is suitable for the medical application such as an additive manufactured medical device or prosthetic appliance.

The silicone compositions according to the invention can also optionally comprise other additives like a standard semi-reinforcing or packing filler, other functional silicone resins such as silicone resin with vinyl group or cyclosiloxanes, non-reactive methyl polysiloxane, pigments and/or colorants, organic solvent or adhesion promoters.

Pigments and colorants can be suitable selected by the person skilled in the art to mimic the color of a human skin. A range of different colors can be provided to meet the needs of the customers.

Regarding semi-reinforcing or packing fillers, non-siliceous or siliceous minerals can be included. Examples of these semi-reinforcing or packing fillers which can be used alone or in combination are ground quartz, diatomaceous earth, carbon black, titanium dioxide, aluminum oxide, hydrated alumina, expanded vermiculite, unexpanded vermiculite, calcium carbonate optionally surface treated by fatty acids, zinc oxide, mica, talc, iron oxide, barium sulfate, and slaked lime. These fillers have a particle size generally comprised between 0.001 µm and 300 µm (micrometers) and a BET surface area of less than 100 m²/g.

Adhesion promoters can further be included within the silicone composition according to the present invention. Typical adhesion promoter compounds are organosilicon compounds comprising a functional adhesion promoting group. For instance, it can be a organosilicon compound comprising :
- one or several hydrolysable groups linked to a silicon atom, for example one or several alkoxy groups linked to a silicon atom; and
- one or several organic groups selected from the group consisting of thiol, urea, isocyanurate, (meth)acrylate, epoxy and alkenyl.

As examples of adhesion promoters, used alone or in combination, it can be cited: vinyltrimethoxysilane (VTMO), 3-glycidoxypropyl-trimethoxysilane (GLYMO), methacryloxypropyltrimethoxysilane (MEMO), [H₂N(CH₂)₃]Si(OCH₂CH₂CH₃)₃, [H₂N(CH₂)₃]Si(OCH₃)₃, [H₂N(CH₂)₃]Si(OC₂H₅)₃, [H₂N(CH₂)₄]Si(OCH₃)₃, [H₂NCH₂CH(CH₃)CH₂CH₂]SiCH₃(OCH₃)₂, [H₂NCH₂]Si(OCH₃)₃, [n-C₄H₉-HN-CH₂]Si(OCH₃)₃, [H₂N(CH₂)₂NH(CH₂)₃]Si(OCH₃)₃, [H₂N(CH₂)₂NH(CH₂)₃]Si(OCH₂CH₂OCH₃)₃, [CH₃NH(CH₂)₂NH(CH₂)₃]Si(OCH₃)₃, [H(NHCH₂CH₂)₂NH(CH₂)₃]Si(OCH₃)₃, HS(CH₂)₃Si(OCH₃)₃, NH₂CONH₂(CH₂)₃Si(OCH₃), or polyorganosiloxane oligomers, containing for instance from 2 to 100 silicon atoms, and containing more than 20% of the above-mentioned organic groups. Organosilicon compounds containing at least one or two alkoxy groups liked to a silicon atom and at least one epoxy group can be also cited.

Adhesion promoters can further be selected from titanium organic compounds, preferably titanium chelate or titanium alkoxide of the general formula Ti(OR)₄ , wherein R is selected from the linear or branched C₁-C₈ alkyl groups, alkoxyalkyl groups and acyl groups. More preferably, the titanium compound can be selected from the groups consisting of butyl titanate, isopropyl titanate, methyl titanate and octyl titanate, even more preferably the titanium compound is butyl titanate (TBOT).

According to the invention, an advantageous combination for the adhesion promoter is as follows:
- vinyltrimethoxysilane (VTMO) and/or methacryloxypropyltrimethoxysilane (MEMO),
- 3-glycidoxypropyltrimethoxysilane (GLYMO), and
- butyltitanate.

Moreover, the adhesion promoter can be an organopolysiloxane compound as disclosed in WO 2017/051084. Said adhesion promoter consists in a linear organopolysiloxane of which all the siloxyl units in the chain are functionalized either with an Si-H unit or with an Si epoxy unit and of which the total number of siloxyl units is between 7 and 30.

Further additives can be for example conservatives, UV-stabilizers, flame retardants, softeners, hardeners, tackifiers, nucleating agents, rheology modifiers, surface additives, flow additives, nanoparticles, antioxidants, toughening agents, thermally insulating particles, electrically conducting particles such as carbon black, graphene, iron, copper, single- and multi-walled carbon nanotubes (SWT, MWT), aluminum, nickel, silver, metallized glass, lead, zinc and alloys, electrically insulating particles, and any combinations or mixtures thereof.

If the 3D-printed silicone object obtained according to the invention is intended to be used in the medical field, then all additives can be appropriately selected among those approved for use in the medical field.

For reasons of storage, the crosslinkable silicone composition according to the present invention can be advantageously presented in the form of an at least two-component system, the mixture of which is capable of crosslinking by polyaddition. The ingredients are then preferably divided into the different parts according to the rules known to a person skilled in the art; in particular the catalyst is separated from the component which comprises the hydrogenosiloxanes.

In the method of post-treatment according to the present invention, the crosslinkable silicone composition is coated on a 3D-printed silicone article.

As used herein, the term "coating" refers to any application form or way which allows the composition to cover sufficiently the area of the silicone article to be post-treated. Examples of coating include dip coating for example dipping into a treatment bath of the silicone composition, spraying coating, curtain coating, spinning coating, painting, brushing or any other manners. Appropriate coating or transfer means can be selected according to the coating technique and to the geometry of the coated 3D-printed article, for example a coating bath, a doctor blade, a roll coater, a brush, a spray gun, etc. The 3D-printed silicone article can be partially or totally coated.

According to an embodiment, the crosslinkable silicone composition is coated with a coating weight of between 50 g/m² and 1000 g/m², preferably between 50 g/m² and 500 g/m², more preferably between 100 g/m² and 300 g/m².

The coating step can be carried out at different temperature, in particular at room temperature.

After coating, the silicone composition can be crosslinked. The crosslinking step can happen at room temperature without any specific action. It is however preferably activated for example by heating, to a temperature comprised between 50°C and 200°C, preferably between 100°C and 150°C. It is up to the person skilled in the art to take account of the maximum heat resistance of the 3D-printed silicone article. The activation means are of the type of those known and appropriate for this purpose, for example thermal or IR radiation. Alternatively, the crosslinking step can be activated by irradiation, preferably by UV radiation. In that case, the person skilled in the art can select a suitable hydrosilylation photocatalyst, and optionally suitable photosensitizers. Any UV light source can be used, such as a LED lamp or mercury lamp as long as it can provide the sufficient energy to crosslink the silicone composition. The UV curing can for instance be conducted for 0.001 s to 30 min, in particular for 0.1 s to 2 min.

According to a preferred embodiment, the post-treatment method according to the invention comprises a step consisting in curing the coated silicone composition by heating to a temperature comprised between 50°C and 200°C, preferably between 100°C and 150°C.

According to an embodiment, the post-treatment method according to the invention can comprise an intermediate step, between the coating step and the curing step by heating, consisting in a settling step, during which the coated 3D-printed silicone article is maintained at room temperature. Said intermediate step can prompt an auto-levelling of the coating. Depending on the geometry of the coated 3D-printed article and on the viscosity of the crosslinkable silicone composition, auto-levelling can be advantageous or detrimental to the final aspect of the surface of the 3D-printed coated silicone article.

The crosslinkable silicone composition disclosed in the present invention is useful for post-treating any 3D-printed silicone elastomer article. Said 3D-printed silicone elastomer article can be obtained by any additive manufacturing method known by the person skilled in the art. It can be obtained from a silicone composition crosslinkable through polyaddition reaction or through polycondensation reaction. Preferably, 3D-printed silicone elastomer article is obtained from a silicone composition crosslinkable through polyaddition reaction.

Without whishing to be bound by any theory, it is believed that the crosslinkable silicone composition according to the present invention, which can crosslink by polyaddition, is particularly suitable for the post-treatment of 3D-printed silicone elastomer article obtained from a silicone composition crosslinkable through polyaddition reaction. The similarity between the coating material and the building material of the article makes it possible to reach good adhesion properties and good mechanical properties.

The present invention further relates to a method for additive manufacturing of a 3D silicone elastomer article comprising the steps of:
a) additive manufacturing a silicone article, and
b) coating the 3D-printed silicone article with the crosslinkable silicone composition comprising a microfiller as defined above.

The additive manufacturing technique used in step (a) can be any technique typically known by the person skilled in the art, and can be preferably an extrusion technique, a jetting technique, or a photopolymerization technique.

According to one preferred embodiment, the step of additive manufacturing a silicone article comprises the steps of:
1) printing a first addition-curable liquid silicone composition on a substrate with a 3D printer selected from an extrusion 3D printer or a material jetting 3D printer to form a first layer,
2) printing a second addition-curable liquid silicone composition on the first or previous layer with the said 3D printer to form a subsequent layer,
3) optionally repeating step 2) with independently selected addition-curable liquid silicone composition for any additional layer needed, and
4) allowing the first and subsequent layers to crosslink, optionally by heating, to obtain a silicone elastomer article.

Some examples of execution are disclosed in the international patent application WO 2020/082359 and WO 2021/211752.

According to another embodiment, the additive manufacturing technique used in step (a) can be a photopolymerization technique. Photopolymerization-based 3D printing techniques are now getting an increased interest. They start from a liquid material either to locally deposit and cure it or to selectively cure it from a liquid vat. Examples of such technologies are UV-Stereolithography (SLA), UV-Digital Light processing (DLP), Continuous Liquid Interface Production (CLIP), UV-extrusion and Inkjet Deposition.

UV-Stereolithography (SLA) is disclosed, for example, in WO 2015/197495. For example, UV-Stereolithography (SLA) uses laser beam which is generally moved in the X-Y (horizontal) plane by a scanner system. Motors guided by information from the generated data source drive mirrors that send the laser beam over the surface.

UV-Digital Light processing (DLP) is disclosed, for example, in WO 2016/181149 and US 2014-0131908. In a UV-Digital Light processing (DLP) a 3D model is sent to the printer, and a vat of liquid polymer is exposed to light from a DLP projector under safelight conditions. The DLP projector displays the image of the 3D model onto the liquid polymer. The DLP projector can be installed under the window which can be made of transparent elastomeric membrane in which the UV light coming from the DLP projector shines through.

Continuous Liquid Interface Production (CLIP, originally Continuous Liquid Interphase Printing) is disclosed, for example, in WO 2014/126837 and WO 2016/140891, which, for example, uses photo polymerization to create smooth-sided solid objects of a wide variety of shapes.

UV-extrusion 3D printing process is disclosed, for example, in WO 2015/107333, WO 2016/109819 and WO 2016/134972. For example, in this process, the material is extruded through a nozzle to print one cross-section of an object, which may be repeated for each layer. An energy source can be attached directly to the nozzle such that it immediately follows extrusion for immediate cure or can be separated from the nozzle for delayed cure. The nozzle or build platform generally moves in the X-Y (horizontal) plane before moving in the Z-axis (vertical) plane once each layer is complete. The UV cure can be immediate after deposition or the plate moves under UV light to give a delay between deposition and UV cure. A support material may be used to avoid extruding a filament material in the air. Some post-processing treatments may be used to improve the quality of the printed surface.

Finally, Inkjet Deposition is disclosed, for example, in WO 2017/40874, WO 2016/071241, WO 2016/134972, WO 2016/188930, WO 2016/044547 and WO 2014/108364, which, for example, uses material jetting printer which has a print head moving around a print area jetting the particular liquid curable composition for example by UV polymerization. The ability of the inkjet nozzle to form a droplet, as well as its volume and its velocity, are affected by the surface tension of the material.

According to this embodiment, the step of additive manufacturing a silicone article comprises the steps of:
1) providing a photocurable composition;
2) exposing the photocurable composition to actinic radiation to form a cured cross-section on a plate or support, and
3) repeating steps 1) and 2) on the former cured cross section with new layer to build up the three-dimensional printed article.

The photocurable composition can be a photocurable liquid silicone composition, preferably a polyaddition curable silicone composition coupled to a photoactivatable catalyst.

Advantageously, no mechanical surface treatments like blasting or polishing for making the surface even or removing the step effect may be needed in the 3D printing or additive manufacturing process according to the present invention.

Use of the post-treated 3D-printed silicone article obtained by the process according to the invention is also provided herein.

In some embodiments, the post-treated 3D-printed silicone article according to the invention can be used in the field of medical materials and/or devices. For example, the article may be suitable for use in a variety of drug delivery devices, implantable devices, medical tubes, stomach catheters, medical balloons, catheter balloons, artificial dialysis machines, blood dialysis machines, implant components, chemical stoppers, O-rings, tubing in peristaltic drug delivery pumps, check valves, resuscitator bulbs, and diaphragm and prosthesis suction cups for limb attachment.

Use of post-treated 3D-printed silicone article according to the invention in an article and/or product, such as a medical device or electronic device described herein, is also provided.

Various embodiments of the present invention can be better understood by reference to the following Examples which are offered by way of illustration. The present invention is not limited to the Examples given herein.

### Examples

### Reference example

The 3D printing has been done using a Delta Tower 3D printer comprising a 2-components Viscotec dosage system equipped with a static mixer for the 2-components silicone composition. The average nozzle diameter used, equal to the thickness of the layer, is 400 µm. The pressure used is 4 bars. The printing speed is adjusted to 5-10 mm/s. No stop is done between each layer printing. No heat or radiation is applied.

Addition-crosslinking silicone composition for the additive manufacturing: AMSil^{®} 20501-50 TRS, commercialized by Elkem Silicones. It is a two-component silicone-based raw material suitable for the Additive Manufacturing of customized as well as complex silicone elastomer technical parts. It crosslinks by polyaddition reaction.

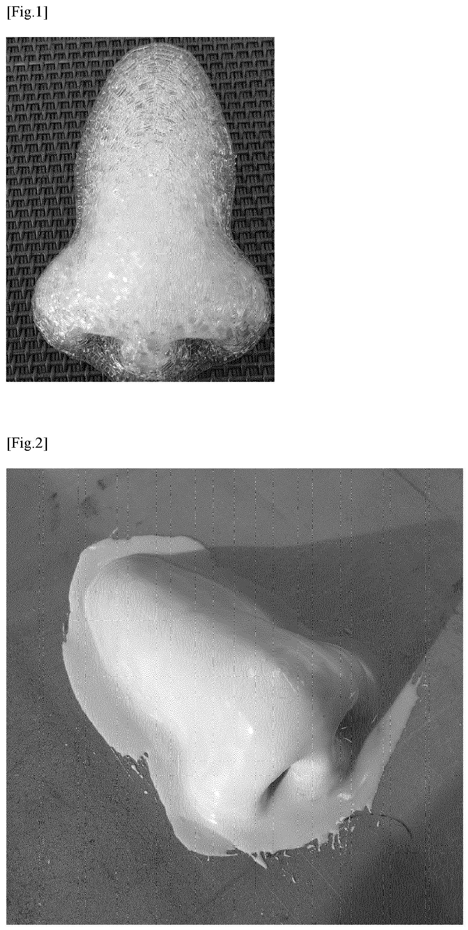

A 3D-printed nose (approx. dimension = 4.1 × 6.6 × 2.4 cm) was obtained, having an obvious step effect on the surface (see Fig.1).

A post-treatment silicone composition (Comp.1) having the composition as disclosed in Table 1 below was prepared.

**Table 1**

| | Comp. 1 |
|---|---|
| A1 = Vinyl terminated polydimethylsiloxane, viscosity: 3500 mPa.s | 65.80% |
| A2 = MD^{Vi}Q branched organopolysiloxane, where the vinyl groups bonded to silicon atoms are carried by the D groups | 21.945% |
| B1 = poly(methylhydrogen)(dimethyl)siloxane with SiH groups in-chain and end-chain (α/ω), viscosity: 25 mPa.s | 10.00% |
| Pt catalyst: Platinum(0)-1,3-divinyl-1,1,3,3-tetramethyldisiloxane (Pt content: 10 wt.%) | 0.02% |
| 1-ethynyl-1-cyclohexanol (ECH) | 0.04% |
| methacryloxypropyltrimethoxysilane (MEMO) | 0.92% |
| 3-glycidoxypropyl-trimethoxysilane (GLYMO) | 0.92% |
| butyl titanate (TBOT) | 0.36% |

The 3D-printed nose was dipped within a bath of the post-treatment silicone composition Comp.1 at room temperature. The post-treated object was subjected to the heat curing under 150°C for 5 min.

The obtained post-treatment coating (thickness around 50-100 µm) successfully adhered to the 3D-printed nose. However, the coating was not sufficient to mask the step-effect.

After 3 successive coatings, the visual aspect of the object improved, but the touch was still not enough good or soft to be qualified as a skin-touch.

### Examples 1-14

To the comparative post-treatment silicone composition (Comp.1) disclosed above, several additives were added according to Table 2:

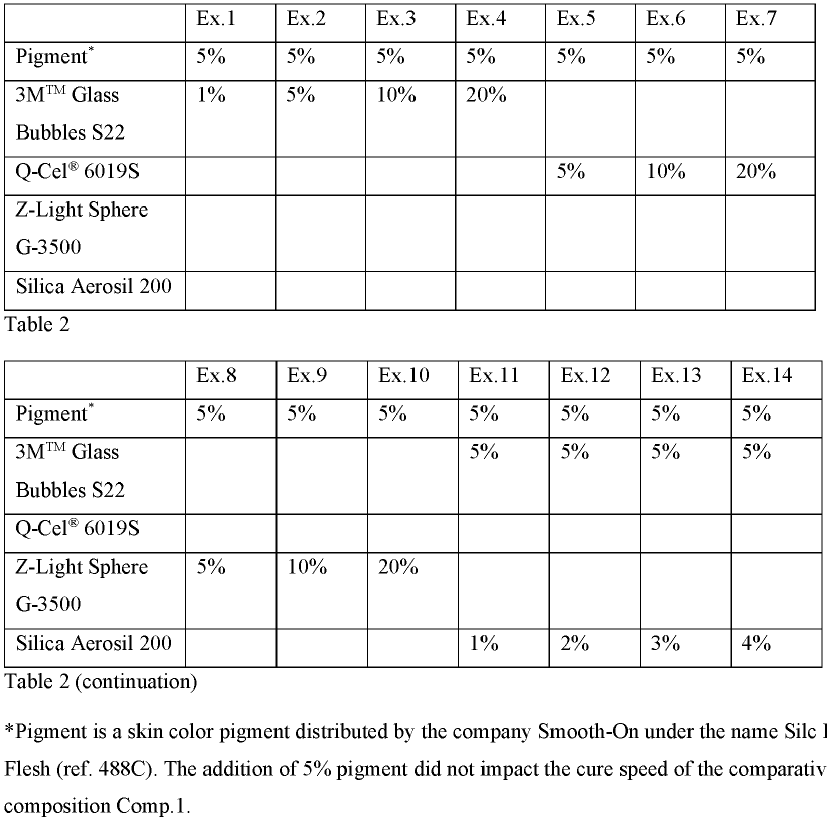

The 3D-printed nose was coated with the post-treatment silicone compositions of Ex.1, Ex.2 and Ex. 5 by brushing in 3 to 5 layers at room temperature. The post-treated objects were subjected to the heat curing at 150°C for 5 min. In all cases, the adhesion between the substrate and coating was successful. The step-effect was visually assessed: When compared to substrate coated with the Comparative composition (Comp. 1), the step-effect to Ex. 1, 2 and 5 has improved. The 3D-printed nose with the post-treatment silicone composition of Ex.2 is shown on Fig.2.

Silicone slabs (9.2cm^{∗}6.6cm^{∗}2mm) were prepared by a casting method with SILBIONE^{®} LSR 4310 composition, a Liquid Silicone Rubbers composition commercially available from Elkem Silicones. SILBIONE^{®} Liquid Silicone Rubbers (LSRs) are two-component platinum-catalyzed silicone elastomers designed for healthcare and medical devices.

The silicone slabs were painted with a brush with the post-treatment silicone compositions at room temperature. The post-treated object was subjected to the heat curing under 150°C for 5 min.

With all post-treatment compositions 1-14, the adhesion between the substrate and coating was successful.

The step-effect was visually assessed: When compared to substrate coated with the Comparative composition (Comp.1), the step-effect to all examples 1-14 has improved. From a visual point of view, the results obtained with 3MTM Glass Bubbles S22 (Ex.1-4 and 11-14) and Q-Cel^{®} 6019S (Ex.5-7) were preferred, because Z-Light Sphere G-3500 filler provided some gray color to the coating, moving away from the appearance of a natural skin.

Addition of Silica Aerosil 200 (Ex. 11-14) avoids phase separation of the coating composition during storage.

Besides, the dynamic coefficient of friction (K_{d}) was measured according to the following method: The coated slab is placed on a sheet of paper. It is loaded with a parallelepipedal mass with a surface area of 40 cm² and a weight of 200 g. The force F_{d} necessary to maintain the displacement of this setup at a speed of displacement of 100 mm/min. The dynamic coefficient of friction is the ratio of this force to the weight applied, i.e. K_{d}=F_{d}/mg. The results of peak force are reported Table 3.

**Table 3**

| | K_{d} |
|---|---|
| Uncoated slab | 2.85 |
| Comp. 1 post-treatment coating | 1.50 |
| Post-treatment coating of Ex.2 | 0.98 |
| Post-treatment coating of Ex.3 | 0.99 |
| Post-treatment coating of Ex. 12 | 1.10 |
| Post-treatment coating of Ex. 13 | 1.12 |

The dynamic coefficient of friction significantly decreased when a post-treatment according to the present invention was performed.

Whereas the difference of dynamic coefficient of friction is not substantial between post-treatment coatings of Ex.2/3 vs Ex. 12/13, the addition of silica filler in Ex.12 & 13 reduced significantly phase separation of the coating composition

### Examples 15-20

To the comparative post-treatment silicone composition (Comp.1) disclosed above, several additives were added according to Table 4:

**Table 4**

| | Ex.15 | Ex.16 | Ex.17 | Ex.18 | Ex.19 | Ex.20 |
|---|---|---|---|---|---|---|
| Pigment | 5% | 5% | 5% | 5% | 5% | 5% |
| (co)polyamide powder Orgasol^{®} 2001 UD NAT 1 | 1% | 5% | 10% | | | |
| (co)poly amide powder Orgasol^{®} 2002 EXD NAT 1 | | | | 1% | 5% | 10% |

### Examples 21-22

3D-printed objects were produced according to the Reference Example disclosed above, except that the final object is not a nose but a slab (6 x 12 cm).

Post-treatment silicone compositions (Ex.21 and Ex.22) having the composition as disclosed in Table 5 below were prepared.

**Table 5**

| | Ex.21 | Ex.22 |
|---|---|---|
| Part A | | |
| A3 = Vinyl terminated polydimethylsiloxane, viscosity: 10,000 mPa.s | 54.59% | 54.59% |
| A4 = MM^{Vi}Q branched organopolysiloxane, where the vinyl groups bonded to silicon atoms are carried by the M groups | 17.96% | 17.96% |
| A5 = Vinyl terminated polydimethylsiloxane gum, consistency at 25°C: 600-900 mm/10 | 2.88% | 2.88% |
| B1 = poly(methylhydrogen)(dimethyl)siloxane with SiH groups in-chain and end-chain (α/ω), viscosity: 25 mPa.s | 4.54% | 4.54% |
| 1-ethynyl-1-cyclohexanol (ECH) | 0.03% | 0.03% |
| Acematt^{®} OK 607 silica microfiller | 20% | |
| Spherilex^{®} 30 A/B silica microfiller | | 20% |
| | | |

| Part B | | |
|---|---|---|
| decamethylcyclopentasiloxane (D5) | 100% | 100% |
| | | |

| Part C | | |
|---|---|---|
| Pt catalyst solution | 100% | 100% |
| | | |
| Mixing ratio Part A : Part B : Part C (by weight) | 100 : 100 : 2 | 100 : 100 : 2 |

The 3D-printed slabs were coated with the post-treatment silicone compositions of Ex.21 and Ex.22 by brushing (1 layer) at room temperature. The post-treated objects were subjected to the heat curing at 150°C for 3 min.

A qualitative assessment of the post-treated slabs has shown that the coating (thickness around 60 µm) strongly adheres to the substrate. No rub-off and no alteration of the coating integrity after stretching (100%) was recorded.

The Coefficient of friction (CoF) was measured with a texture analyzer equipped with a CoF sled according to the standard ASTM D1894 / ISO 8295. The results of peak force and sliding force are reported Table 6.

**Table 6**

| | Peak Force (kg) | Sliding force (kg) |
|---|---|---|
| Not post-treated slab | 2.48 | 0.5 |
| Post-treatment coating of Ex.21 | 0.6 | 0.5 |
| Post-treatment coating of Ex.22 | 0.85 | 0.5 |

## Claims

1. Method of post-treatment of a 3D-printed silicone article, said method comprising coating the 3D-printed silicone article with a crosslinkable silicone composition comprising a microfiller.

2. Method of post-treatment according to Claim 1, wherein the microfiller is selected from the group of powders comprising substantially spherical particles.

3. Method of post-treatment according to Claim 1 or Claim 2, wherein the microfiller is selected from the group consisting of low-density hollow fillers, powders of (co)polyamides, silica or glass microfillers and spherical silicone resin particles.

4. Method of post-treatment according to any of Claim 1 to Claim 3, wherein the crosslinkable silicone composition comprises a low-density hollow filler, having preferably a true density below 1 g/cm³, more preferably below 0.8 g/cm³, even more preferably below 0.5 g/cm³, and even more preferably between 0.01 g/cm³ and 1 g/cm³.

5. Method of post-treatment according to Claim 4, wherein the low-density hollow filler is hollow microspheres, preferably selected from mineral hollow microsphere and organic hollow microsphere.

6. Method of post-treatment according to Claim 4 or Claim 5, wherein the low-density hollow filler is hollow glass microspheres.

7. Method of post-treatment according to any of Claim 1 to Claim 6, wherein the amount of microfiller in the crosslinkable silicone composition is in the range from 0.1 wt.% to 50 wt.%, preferably 1 wt.% to 20 wt.%, more preferably 2 wt.% to 15 wt.%, and even more preferably 5 wt.% to 10 wt.%, with respect to the total weight of the crosslinkable silicone composition.

8. Method of post-treatment according to any of Claim 1 to Claim 7, wherein the crosslinkable silicone composition comprises:
(A) at least one organopolysiloxane compound A comprising, per molecule, at least two alkenyl radicals bonded to silicon atoms,
(B) at least one organohydrogenopolysiloxane compound B comprising, per molecule, at least two hydrogen atoms bonded to an identical or different silicon atom,
(C) at least one polyaddition catalyst C, preferably a polyaddition catalyst consisting of at least one metal or compound from the platinum group,
(D) at least said microfiller D;
(E) optionally, at least one crosslinking inhibitor E;
(F) optionally, at least one reinforcing filler F;
(G) optionally, at least one solvent G.

9. Method of post-treatment according to any of Claim 1 to Claim 8, wherein the crosslinkable silicone composition is coated on the 3D-printed silicone article by dip coating, spraying coating, curtain coating, spinning coating, painting, or brushing.

10. Method of post-treatment according to any of Claim 1 to Claim 9, wherein the crosslinkable silicone composition is coated with a coating weight of between 50 g/m² and 1000 g/m², preferably between 50 g/m² and 500 g/m², more preferably between 100 g/m² and 300 g/m².

11. Method for additive manufacturing of a 3D silicone elastomer article comprising the steps of:
a) additive manufacturing a silicone article, and
b) coating the 3D-printed silicone article with a crosslinkable silicone composition comprising a microfiller.

12. Method for additive manufacturing of a 3D silicone elastomer article according to Claim 11, wherein the additive manufacturing technique used in step (a) is an extrusion technique, a jetting technique, or a photopolymerization technique.

13. 3D silicone elastomer article obtained or obtainable by the method according to Claim 11 or Claim 12, wherein said 3D silicone elastomer article comprises a coating with a crosslinkable silicone composition comprising a microfiller.

14. 3D silicone elastomer article according to Claim 13, wherein said 3D silicone elastomer article is a medical material and/or a medical device.

15. Use of a crosslinkable silicone composition comprising a microfiller as post-treatment composition for the post-treatment of a 3D printed silicone article.
